# EUROPEAN PATENT SPECIFICATION

(11) **EP 3 939 501 B1**
(45) Date of publication and mention of the grant of the patent: **15.01.2025**
(21) Application number: 20208923.1
(22) Date of filing: 20.11.2020
(51) Int. Cl.: A61B 5/024, A61B 5/1455, A61B 5/00

(54) **BIOSIGNAL SENSOR**
BIOSIGNALSENSOR
CAPTEUR DE BIOSIGNAUX

(30) Priority: 13.07.2020 KR 20200086421
(43) Date of publication of application: 19.01.2022
(73) Proprietor: Samsung Electronics Co., Ltd., Suwon-si, Gyeonggi-do 16677 (KR)
(72) Inventor: LEE, Gae Hwang, Seongnam-si, Gyeonggi-do (KR); KANG, Hyun Bum, Yongin-si, Gyeonggi-do (KR); YUN, Youngjun, Seongnam-si, Gyeonggi-do (KR); CHUNG, Jong Won, Hwaseong-si, Gyeonggi-do (KR)
(74) Representative: Elkington and Fife LLP

(56) References cited:
- WO-A1-2017/080869
- WO-A1-2020/074497
- WO-A1-2020/137148
- US-A1- 2014 350 365
- US-A1- 2016 013 223

## Description

### FIELD OF THE INVENTION

Biosignal sensors, sensor arrays, and sensor systems are disclosed.

### BACKGROUND OF THE INVENTION

Recently, research on skin-attachable devices for obtaining bio-information by attaching same to the skin is in progress. Such skin-attachable devices include biosensors for obtaining bio-information. For example, a photoplethysmography (PPG) sensor may obtain a PPG signal from a user, and by analyzing the PPG signal, bio-information such as a user's blood pressure, arrhythmia, heart rate, and/or oxygen saturation may be obtained.

Document US 2014/0350365 A1 discloses a measurement device for measuring a biological component, the measurement device including a measurement unit configured to have a light source unit, a detection unit and a polarization control unit, the polarization unit being provided in a position between the light source unit and a living body and between the detection unit and the living body.

Document WO 2017/080869 A1 discloses a photoplethysmography device comprising a light source, a light sensor and an optical blocking arrangement, the PPG device further comprising a polarization filter and a silicone protection layer.

Document WO 2020/074497 A1 discloses an optoelectric sensor comprising a radiation-emitting semiconductor region, a radiation-detecting semiconductor region, a first polarization filter and a second polarization filter.

Document US 2016/0013223 A1 discloses an optical module including a light source, light detector and first and second polarizers embedded in an enclosure.

### SUMMARY OF THE INVENTION

The invention is defined by the appended claims 1-13.

Some example embodiments provide a biosignal sensor capable of improving performance of obtaining bio-information by reducing noise caused by a change in position between a skin and a biosensor due to motion.

Some example embodiments provide a sensor array including the biosignal sensor.

Some example embodiments provide a sensor system including the biosignal sensor or the sensor array.

According to an aspect of the invention, there is provided a biosignal sensor according to claim 1.

In some embodiments, the first polarizer may at least partially overlap with the light source in a first direction along which the light source is configured to emit light, and the second polarizer may at least partially overlap with the light sensor in the first direction.

In an embodiment, a polarization direction of the second polarizer may be different from a polarization direction of the first polarizer.

In an embodiment, an angle between the polarization direction of the second polarizer and the polarization direction of the first polarizer may be about 30 degrees to about 90 degrees.

In an embodiment, a polarization direction of the second polarizer may be perpendicular to the polarization direction of the first polarizer.

In an embodiment, the second polarizer may be configured to selectively block light in a polarization direction parallel to a polarization direction of the linearly polarized light passing through the first polarizer, and selectively transmit light in a separate polarization direction different from the polarization direction of the linearly polarized light passing through the first polarizer.

In an embodiment, the first polarizer may include a plurality of first patterns extending in a first direction. The second polarizer may include a plurality of second patterns extending in a second direction, the second direction being different from the first direction.

In an embodiment, the plurality of first patterns and the plurality of second patterns may be embedded within the light-transmitting layer.

In an embodiment, the first direction and the second direction may be perpendicular to each other.

In an embodiment, the biosignal sensor may further include a phase retarder between the light source and the first polarizer.

According to the invention, the light-transmitting layer includes a plurality of first regions and a second region between the adjacent first regions, the first regions having a first elastic modulus, the second region having a second elastic modulus that is lower than the first elastic modulus. The light source and the light sensor are each on a separate first region of the plurality of first regions.

In an embodiment, the biosignal sensor may be a skin-attachable photoplethysmography (PPG) sensor.

A sensor array may include the biosignal sensor.

The sensor array may include a plurality of unit element groups, and each unit element group of the plurality of unit element groups may include at least one light source, at least one light sensor, at least one first polarizer, and at least one second polarizer.

In an embodiment, each unit element group may include a first light source configured to emit light in a first wavelength spectrum and a second light source configured to emit light in a second wavelength spectrum, the second wavelength spectrum being longer than the first wavelength spectrum. The first polarizer may include a third polarizer overlapped with the first light source, and a fourth polarizer overlapped with the second light source. A polarization direction of the third polarizer and a polarization direction of the fourth polarizer may be in parallel.

In an embodiment, the polarization direction of the third polarizer and the polarization direction of the fourth polarizer may be perpendicular to the polarization direction of the second polarizer.

In an embodiment, the each unit element group may further include a pressure sensor.

A sensor system may include the biosignal sensor.

A sensor system may include the sensor array.

A noise caused by a positional change between the skin and the biosensor due to motion may be reduced and thus performance may be improved.

### BRIEF DESCRIPTION OF THE DRAWINGS

FIG. 1 is a plan view showing an example of a biosignal sensor according to some example embodiments,
FIG. 2 is a cross-sectional view of an example of the biosignal sensor of FIG. 1 taken along line II-II' in FIG. 1, according to some example embodiments,
FIG. 3 is a plan view showing an example of a first polarizer, according to some example embodiments,
FIG. 4 is a plan view showing an example of a second polarizer, according to some example embodiments,
FIG. 5 is a schematic view showing an example of a propagation path of light in the biosignal sensor of FIGS. 1 and 2, according to some example embodiments,
FIG. 6 is a cross-sectional view of an example of the biosignal sensor of FIG. 1 taken along line II-II' in FIG. 1, according to some example embodiments,
FIG. 7 is a schematic view showing a change in polarization of the biosignal sensor of FIG. 6, according to some example embodiments,
FIG. 8 is a plan view showing another example of a biosignal sensor according to some example embodiments,
FIG. 9 is a cross-sectional view showing the biosignal sensor of FIG. 8 taken along line IX-IX' in FIG. 8, according to some example embodiments,
FIG. 10 is a schematic view showing an example arrangement of a biosignal sensor array according to some example embodiments,
FIG. 11 is a schematic view showing a portion of the biosignal sensor array of FIG. 10, according to some example embodiments,
FIG. 12 is a schematic view showing an arrangement of another example of a biosignal sensor array according to some example embodiments,
FIG. 13 is a schematic view showing a portion of the biosignal sensor array of FIG. 12, according to some example embodiments,
FIG. 14 is a schematic view showing an example of a sensor system according to some example embodiments,
FIG. 15 is a graph showing biosignal changes of a biosignal sensor according to Example 4,
FIG. 16 is a graph showing biosignal changes of the biosignal sensor according to Example 5,
FIG. 17 is a graph showing biosignal changes of a biosignal sensor according to Example 4, and
FIG. 18 is a graph showing biosignal changes of a biosignal sensor according to Comparative Example 4.

### DETAILED DESCRIPTION

Hereinafter, example embodiments are described in detail so that those of ordinary skill in the art can easily implement them. However, the structures that are actually applied may be implemented in various different forms, and is not limited to the example embodiments described herein. Yet, the present invention is defined by the appended claims 1-13.

In the drawings, the thickness of layers, films, panels, regions, etc., are exaggerated for clarity. It will be understood that when an element such as a layer, film, region, or substrate is referred to as being "on" another element, it may be directly on the other element or intervening elements may also be present. In contrast, when an element is referred to as being "directly on" another element, there are no intervening elements present. An element that is "on" another element may be above or beneath the other element.

Hereinafter, a biosignal sensor according to some example embodiments is described.

The biosignal sensor is a biosensor capable of detecting a biosignal in a living body temporarily or in real time. The biosignal may be, for example, a blood flow rate, a change in oxygen distribution, and/or an electrocardiogram.

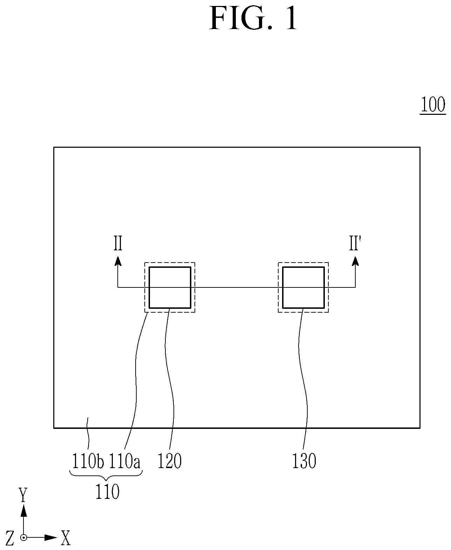

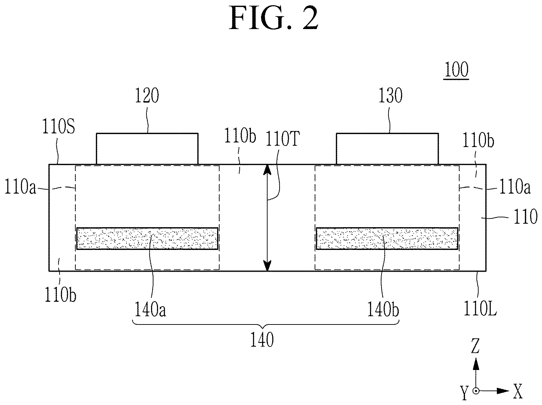

FIG. 1 is a plan view showing an example of a biosignal sensor according to some example embodiments, and FIG. 2 is a cross-sectional view showing an example of the biosignal sensor of FIG. 1 taken along line II-II' in FIG. 1, according to some example embodiments.

Referring to FIGS. 1 and 2, the biosignal sensor 100 according to some example embodiments includes a light-transmitting layer 110, a light-emitting element 120, and a photo-detective element 130, and a polarizer 140.

The light-transmitting layer 110 may be disposed under the light-emitting element 120 and the photo-detective element 130 to support the light-emitting element 120 and the photo-detective element 130. Restated, the light-emitting element 120 and the photo-detective element 130 may be on the light-transmitting layer 110, for example directly on (e.g., in direct contact with) the upper surface 110S of the light-transmitting layer 110. The light-transmitting layer 110 may be, for example, a support substrate or may be formed on a separate support substrate (not shown). When a separate support substrate is included, the support substrate may be a stretchable substrate. In some example embodiments, the light-transmitting layer 110 may be absent. In some example embodiments, the light-emitting element 120 and/or the photo-detective element 130 may be at least partially embedded within the light-transmitting layer 110.

The light-transmitting layer 110 may be configured to transmit light, and may have for example, light transmittance of greater than or equal to about 70%, greater than or equal to about 75%, greater than or equal to about 80%, greater than or equal to about 85%, greater than or equal to about 90%, greater than or equal to about 95%, greater than or equal to about 97%, greater than or equal to about 98%, or greater than or equal to about 99%. The light-transmitting layer 110 may be disposed in a direction in which light is emitted from the light-emitting element 120 and in a direction in which light is flowed into the photo-detective element 130.

Such a direction may be a thickness direction of the light-transmitting layer 110 (e.g., a direction of the thickness 110T of the light-transmitting layer 110 between opposite surfaces 110S, 110L), and thus a direction which is perpendicular to at least the upper surface 110S. As shown, such a direction (e.g., thickness direction) may be the Z direction.

For example, the light-transmitting layer 110 may be disposed closer to skin or the living body (e.g., blood vessel) to be detected than the light-emitting element 120 and the photo-detective element 130. Restated, the light-emitting element 120 and the photo-detective element 130 may be distal from a lower surface 110L of the light-transmitting layer, in relation to the upper surface 110S.

As shown in at least FIG. 2, the light-emitting element 120 and the photo-detective element 130 may be adjacent to each other, and spaced apart (e.g., isolated from direct contact with each other) in a first direction (e.g., the X direction and/or Y direction). The light-emitting element 120 may be configured to emit light in a second direction (e.g., the Z direction) that is different from the first direction, and the photo-detective element 130 may be configured to receive and thus detect light incident on the photo-detective element 130 in the same second direction (e.g., the Z direction).

The light-transmitting layer 110 may be a stretchable light-transmitting layer, and the stretchable light-transmitting layer may respond flexibly to external forces or external motions such as twisting, pressing, and pulling, and may be easily restored to its original state. The light-transmitting layer 110 may include a stretchable material such as an elastomer, and the stretchable material may include an organic elastomer, an organic/inorganic elastomer, an inorganic elastomer-like material, or a combination thereof. The organic elastomer or the organic/inorganic elastomer may be, for example, substituted or unsubstituted polyorganosiloxane such as polydimethylsiloxane; an elastomer including substituted or unsubstituted butadiene moiety such as styrene-ethylene-butylene-styrene; an elastomer including a urethane moiety; an elastomer including an acrylic moiety; an elastomer including an olefin moiety; or a combination thereof, but is not limited thereto. The inorganic elastomer-like material may include an elastic ceramic, an elastic solid metal, liquid metal, or a combination thereof, but is not limited thereto.

The light-transmitting layer 110 may include regions having different stiffness, for example, first regions 110a having relatively high stiffness and a second region 110b having a relatively low stiffness than the first region 110a. Herein, the stiffness may indicate a degree of resistance to deformation when a force is applied from the outside. Relatively high stiffness may mean that the resistance to deformation is relatively large, so that deformation is small while relatively low stiffness may mean that the resistance to deformation is relatively small, so that the deformation is large.

The stiffness may be evaluated from an elastic modulus, and a high elastic modulus may mean high stiffness and a low elastic modulus may mean low stiffness. Accordingly, the first regions 110a may each have a first elastic modulus and the second region 110b may have a second elastic modulus that is lower than the first elastic modulus. The elastic modulus may be, for example, a Young's modulus. A difference between elastic moduli of the first region 110a and the second region 110b of the light-transmitting layer 110 may be about 100 times or more, and the elastic modulus of the first region 110a may be about 100 times higher than the elastic modulus of the second region 110b. The difference between the elastic modulus of the first region 110a and the second region 110b may be about 100 to 100,000 times within the above range, and the elastic modulus of the first region 110a may be about 100 times to about 100,000 times higher than the elastic modulus of the second region 110b, but is not limited thereto. For example, the elastic modulus of the first region 110a may be about 10⁷ Pa to about 10¹² Pa, and the elastic modulus of the second region 110b may be greater than or equal to about 10² Pa and less than about 10⁷ Pa, but is not limited thereto.

Elongation rates of the first region 110a and the second region 110b of the light-transmitting layer 110 may be different due to the aforementioned difference in stiffness, and the elongation rate of the second region 110b may be higher than the elongation rate of the first region 110a. Herein, the elongation rate may be a percentage of the length change that is increased to a breaking point with respect to the initial length. For example, the elongation rate of the first region 110a of the light-transmitting layer 110 may be less than or equal to about 5%, within the range, about 0% to about 5%, about 0% to about 4%, about 0% to about 3%, about 0% to about 2%, about 0% to about 1%, about 0.5% to about 5%, about 0.5% to about 4%, about 0.5% to about 3%, about 0.5% to about 2%, or about 1% to about 2%. For example, the elongation rate of the second region 110b of the light-transmitting layer 110 may be greater than or equal to about 10%, within the range, about 10% to about 300%, about 10% to about 200%, about 10% to about 100%, about 10% to about 90%, about 10% to about 80%, about 10% to about 70%, about 10% to about 60%, about 10% to about 50%, about 10% to about 40%, about 20% to about 70%, about 20% to about 60%, about 20% to about 50%, or about 20% to about 40%.

A plurality of first regions 110a of the light-transmitting layer 110 may have an island-shape separated from (e.g., isolated from direct contact with) each other, and the light-emitting element 120 and the photo-detective element 130 which are described later are disposed on each first region 110a of the light-transmitting layer 110.

The light-emitting element 120 and the photo-detective element 130 may be each on and/or at least partially embedded in a separate first region 110a of the plurality of first regions 110a of the light-transmitting layer 110. In some example embodiments, the first regions 110a may each be sized to have dimensions in the X direction and Y direction (e.g., the one or more directions in which the light-transmitting layer 110 extends and/or the one or more directions parallel to at least the upper surface 110S of the light-transmitting layer 110) that are the same as, or different from, the dimensions, in the X direction and/or the Y direction, of the corresponding light-emitting element 120 or the photo-detective element 130 that are on and/or in said first regions 110a. However, example embodiments are not limited thereto.

For example, as shown in FIG. 2, in some example embodiments, the first regions 110a may each have a width (e.g., dimension in the X direction) that is the same as a width (e.g., dimension in the X direction) of the corresponding polarizers 140 included in said first regions 110a and which overlap a corresponding light-emitting element 120 or photo-detective element 130 in the Z direction, such that the dimensions of the first regions 110a in the X direction may be different from (e.g., greater than or less than) the dimensions in the X direction of the corresponding light-emitting element 120 or the photo-detective element 130 on or at least partially embedded in said first regions 110a.

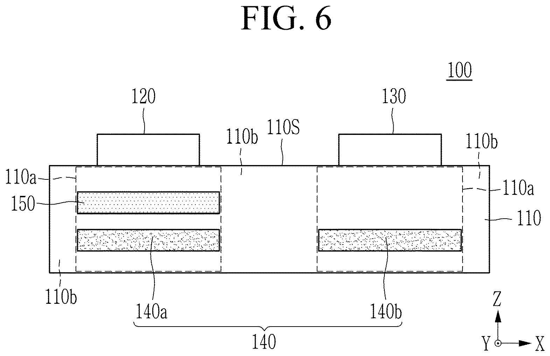

The above description with regard to the X direction may apply equally or alternatively to the Y direction. Thus, it will be understood that the dimensions of a first region 110a, in the X direction and/or Y direction may be the same as or different from (e.g., greater than or less than) the dimensions, in the X direction and/or Y direction, of the corresponding light-emitting element 120 or photo-detective element 130 on or at least partially embedded in said first region 110a. In another example, the dimensions of a first region 110a, in the X direction and/or Y direction may be the same as or different from (e.g., greater than or less than) the dimensions, in the X direction and/or Y direction, of the corresponding polarizer 140 included in said first region 110a. Additionally, with reference to FIG. 6 below, the dimensions of a first region 110a, in the X direction and Y direction, may be the same as or different from (e.g., greater than or less than) the dimensions, in the X direction, of a corresponding phase retarder 150 included in said first region 110a.

The second region 110b of the light-transmitting layer 110 may be a region other than the plurality of first regions 110a and may be entirely continuously connected. The second region 110b of the light-transmitting layer 110 may be a region providing stretchability and due to its relatively low stiffness and high elongation rate, it may flexibly respond to external forces or external motions such as twisting and pulling, and may be easily restored to its original state.

For example, the first region 110a and the second region 110b of the light-transmitting layer may have different shapes. For example, the first region 110a of the light-transmitting layer 110 may be flat and the second region 110b may include a two-dimensional or three-dimensional stretchable structure. The two-dimensional or three-dimensional stretchable structure may have, for example, a wavy shape, a wrinkle shape, a pop-up shape, or a non-coplanar mesh shape, but is not limited thereto.

For example, the first region 110a and the second region 110b of the light-transmitting layer 110 may include different materials. For example, the first region 110a of the light-transmitting layer 110 may include an inorganic material, an organic material, and/or an organic/inorganic material having relatively high stiffness and a low elongation rate, and the second region 110b of the light-transmitting layer 110 may include an inorganic material, an organic material, and/or an organic/inorganic material having a relatively low stiffness and high elongation rate. For example, the first region 110a of the light-transmitting layer 110 may include an organic material such as polycarbonate, polymethylmethacrylate, polyethyleneterephthalate, polyethylenenaphthalate, polyimide, polyamide, polyamideimide, polyethersulfone, or a combination thereof, a carbon structure such as diamond carbon, and the second region 110b of the light-transmitting layer 110 may include an organic or organic/inorganic elastomer such as a substituted or unsubstituted polyorganosiloxane such as polydimethylsiloxane, an elastomer including a substituted or unsubstituted butadiene moiety such as styrene-ethylene-butylene-styrene, an elastomer including a urethane moiety, an elastomer including an acrylic moiety, an elastomer including an olefin moiety, or a combination thereof; an inorganic elastomer-like material such as ceramic, a solid metal, a liquid metal, or a combination thereof, but they are not limited thereto.

For example, the first region 110a and the second region 110b of the light-transmitting layer 110 may be formed with the same material (e.g., may be separate parts of a single piece of material), and may have different stiffness by being exposed to different conditions such as polymerization degrees and/or curing degrees. For example, the light-transmitting layer 110 may be a single piece of material that has the first region 110a having a relatively high stiffness and the second region 110b having a relatively low stiffness which are formed by varying the polymerization degrees, types and contents of curing agents, and/or curing temperatures, based on polydimethylsiloxane.

In this way, the light-transmitting layer 110 includes a first region 110a having a relatively high stiffness and a low elongation rate, and a second region 110b having a relatively low stiffness and a high elongation rate, and includes a light-emitting element 120 and a photo-detective element which are disposed in the first region 110a, which will be described later, and thereby even when a large external force or motion is applied to the light-transmitting layer 110, the light-emitting element 120 and photo-detective element 130 in the first region 110a may receive relatively less strain and thus, the light-emitting element 120 and photo-detective element 130 are prevented from being damaged or destroyed by excessive strain.

The light-emitting element 120 and photo-detective element 130 are disposed on the light-transmitting layer 110. The light-emitting element 120 and the photo-detective element 130 are arranged in parallel in a first direction (e.g., X direction and/or Y direction) at a particular (or, alternatively, predetermined) interval.

The light-emitting element 120, also referred to herein interchangeably as a light emitter, light source, or the like, may be configured to emit light in a particular (or, alternatively, predetermined) wavelength spectrum, and may include, for example, an inorganic light emitting diode (e.g., LED), an organic light emitting diode (OLED), an organic/inorganic light emitting diode, or a micro light emitting diode. The light-emitting element 120 may include, for example, a pair of electrodes and a light emitting layer between the pair of electrodes.

For example, one electrode of the pair of electrodes may be a light transmitting electrode and the other electrode of the pair of electrodes may be a reflecting electrode, for example, an electrode disposed close to the light-transmitting layer 110 may be a light transmitting electrode. The light-transmitting electrode may include for example at least one of an oxide conductor, a carbon conductor, and a metal thin film. The oxide conductor may include for example at least one of indium tin oxide (ITO), indium zinc oxide (IZO), zinc tin oxide (ZTO), aluminum tin oxide (AlTO), and aluminum zinc oxide (AZO), the carbon conductor may include at least one of graphene and carbon nanostructures, and the metal thin film may be a very thin film including aluminum (Al), magnesium (Mg), silver (Ag), gold (Au), an alloy thereof, or a combination thereof. The reflecting electrode may include a reflective conductor such as a metal and may include, for example aluminum (Al), silver (Ag), gold (Au), or an alloy thereof. For example, the pair of electrodes may be stretchable electrodes, and the stretchable electrodes may include, for example, a stretchable conductor, or may have a stretchable shape such as a wavy, wrinkled, pop-up, or non-planar mesh shape.

For example, the light emitting layer may include an emitter such as an organic light emitting material, a quantum dot, and/or perovskite, but is not limited thereto. The organic light-emitting material may include, for example, perylene or a derivative thereof, rubrene or a derivative thereof, 4-(dicyanomethylene)-2-methyl-6-[p-(dimethylamino)styryl]-4H-pyran or a derivative thereof, cumarin or a derivative thereof, carbazole or a derivative thereof, an organometallic compound including Pt, Os, Ti, Zr, Hf, Eu, Tb, Tm, Rh, Ru, Re, Be, Mg, Al, Ca, Mn, Co, Cu, Zn, Ga, Ge, Pd, Ag and/or Au, or a combination thereof. The quantum dot may include, for example, a Group II-VI semiconductor compound, a Group III-V semiconductor compound, a Group IV-VI semiconductor compound, a Group IV semiconductor element or compound, a Group I-III-VI semiconductor compound, a Group I-II-IV-VI semiconductor compound, a Group II-III-V semiconductor compound, or a combination thereof. The Group II-VI semiconductor compound may be for example a binary element of CdSe, CdTe, ZnS, ZnSe, ZnTe, ZnO, HgS, HgSe, HgTe, MgSe, MgS, or a combination thereof; a ternary element of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, or a combination thereof; a quaternary element of ZnSeSTe, HgZnTeS, CdZnSeS, CdZnSeTe, CdZnSTe, CdHgSeS, CdHgSeTe, CdHgSTe, HgZnSeS, HgZnSeTe, HgZnSTe, or a combination thereof; or a combination thereof, but is not limited thereto. The Group III-V semiconductor compound may be for example a binary element of GaN, GaP, GaAs, GaSb, AlN, AlP, AlAs, AlSb, InN, InP, InAs, InSb, or a combination thereof; a ternary element of GaNP, GaNAs, GaNSb, GaPAs, GaPSb, AlNP, AlNAs, AlNSb, AlPAs, AlPSb, InNP, InNAs, InNSb, InPAs, InPSb, or a combination thereof; a quaternary element of GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, or a combination thereof; or a combination thereof, but is not limited thereto. The Group IV-VI semiconductor compound may be for example a binary element of SnS, SnSe, SnTe, PbS, PbSe, PbTe, or a combination thereof; a ternary element of SnSeS, SnSeTe, SnSTe, PbSeS, PbSeTe, PbSTe, SnPbS, SnPbSe, SnPbTe, or a combination thereof; a quaternary element of SnPbSSe, SnPbSeTe, SnPbSTe, or a combination thereof; or a combination thereof, but is not limited thereto. The Group IV semiconductor element or compound may be for example a singular element semiconductor of Si, Ge, or a combination thereof; a binary element semiconductor of SiC, SiGe, or a combination thereof; or a combination thereof, but is not limited thereto. The Group I-III-VI semiconductor compound may be for example selected from CuInSe₂, CuInS₂, CuInGaSe, CuInGaS, or a combination thereof, but is not limited thereto. The Group I-II-IV-VI semiconductor compound may be for example CuZnSnSe, CuZnSnS, or a combination thereof, but is not limited thereto. The Group II-III-V semiconductor compound may include for example InZnP, but is not limited thereto. The perovskite may include, for example, CH₃NH₃PbBr₃, CH₃NH₃Pbl₃, CH₃NH₃SnBr₃, CH₃NH₃SnI₃, CH₃NH₃Sn₁₋ₓPbₓBr₃, CH₃NH₃Sn₁₋ₓPbₓI₃, HC(NH₂)₂PbI₃, HC(NH₂)₂SnI₃, (C₄H₉NH₃)₂PbBr₄, (C₆H₅CH₂NH₃)₂PbBr₄, (C₆H₅CH₂NH₃)₂PbI₄, (C₆H₅C₂H₄NH₃)₂PbBr₄, (C₆H₁₃NH₃)₂(CH₃NH₃)ₙ₁iPbₙI₃ₙ₊₁, or a combination thereof. The light emitting layer may be configured to emit at least a portion of light in a visible wavelength spectrum and/or an infrared wavelength spectrum, for example light in any one of a blue wavelength spectrum, a green wavelength spectrum, a red wavelength spectrum, or an infrared wavelength spectrum, for example light in any one of a green wavelength spectrum, a red wavelength spectrum, or an infrared wavelength spectrum, for example light in a green wavelength spectrum. The pair of electrodes may be stretchable electrodes, and the light emitting layer may be a stretchable light emitting layer, and accordingly, the light-emitting element 120 may be, for example, a stretchable light-emitting element.

The photo-detective element 130, also referred to herein interchangeably as a photodetector, light sensor, or the like, may be configured to absorb light in a particular (or, alternatively, predetermined) wavelength spectrum, and may include, for example, an inorganic photodiode or an organic photoelectric conversion element. The photo-detective element 130 may include, for example, a pair of electrodes and a photoelectric conversion layer between the electrodes. For example, one electrode of the pair of electrodes may be a light-transmitting electrode and the other electrode of the pair of electrodes may be a reflecting electrode, for example, an electrode disposed close to the light-transmitting layer 110 may be a light-transmitting electrode. The light-transmitting electrode may include for example at least one of an oxide conductor, a carbon conductor, and a metal thin film. The oxide conductor may include for example at least one of indium tin oxide (ITO), indium zinc oxide (IZO), zinc tin oxide (ZTO), aluminum tin oxide (AlTO), and aluminum zinc oxide (AZO), the carbon conductor may include at least one of graphene and carbon nanostructures, and the metal thin film may be a very thin film including aluminum (Al), magnesium (Mg), silver (Ag), gold (Au), an alloy thereof, or a combination thereof. The reflecting electrode may include a reflective conductor such as a metal and may include, for example aluminum (Al), silver (Ag), gold (Au), or an alloy thereof. For example, the pair of electrodes may be stretchable electrodes, and the stretchable electrodes may include, for example, a stretchable conductor, or may have a stretchable shape such as a wavy, wrinkled, pop-up, or non-planar mesh shape. As an example, the photoelectric conversion layer may include, for example, an inorganic semiconductor, an organic semiconductor, and/or an organic/inorganic semiconductor, and may include, for example, a p-type semiconductor and an n-type semiconductor forming a pn junction. As an example, the photoelectric conversion layer may include, for example, Si, GaN, AlGaN, GaAsP, metal phthalocyanine, rubrene, pentacene, fullerene, thiophene, quinoid, a derivative thereof, or a combination thereof. As an example, the photoelectric conversion layer may be a stretchable photoelectric conversion layer. The photo-detective element 130 may be, for example, a stretchable photo-detective element.

The light emitted from the light-emitting element 120 may pass through the light-transmitting layer 110 and be scattered and reflected by the target portion (hereinafter referred to as a 'target') of a living body such as a blood vessel, and the scattered and reflected light passes through the light-transmitting layer 110 again and may be absorbed in the photo-detective element 130 to obtain a biosignal.

The polarizer 140 includes a first polarizer 140a and a second polarizer 140b.

The first polarizer 140a may be disposed at a position through which light emitted from the light-emitting element 120 passes, and may be disposed to be at least partially overlapped with the light-emitting element 120 in the thickness direction of the light-transmitting layer 110, which is a direction along which the thickness 110T of the light-transmitting layer extends (e.g., the Z direction), which also may be a direction that extends perpendicular to an upper surface 110S of the light-transmitting layer 110. For example, the first polarizer 140a may be embedded in the light-transmitting layer 110. For example, the first polarizer 140a may be attached to the light-emitting element 120. For example, the first polarizer 140a may be disposed to be at least partially overlapped with the light-emitting element 120 on the light-transmitting layer 110 in the direction along which the light-emitting element 120 is configured to emit light (e.g., L1). For example, the light-emitting element 120 may be configured to emit light in a first direction (e.g., the Z direction). The first polarizer 140a may be positioned to at least partially overlap the light-emitting element 120 in the first direction (e.g., the Z direction) that the light-emitting element 120 is configured to emit light (e.g., L1), such that the biosignal sensor 100 is configured to cause the light-emitting element 120 to emit light (e.g., L1) along the first direction (e.g., the -Z direction) such that the emitted light (e.g., L1) subsequently passes through, and is linearly polarized by, the first polarizer 140a. The first polarizer 140a may be configured to linearly polarize the light (e.g., L1) emitted from the light-emitting element 120, such that the first polarizer 140a selectively transmits the linearly polarized light (e.g., L2), such that the biosignal sensor 100 is configured, based on the relative positioning of the light-emitting element 120 and the first polarizer 140a, to cause at least some, or all, of the light emitted from the light-emitting element 120 to pass through the first polarizer 140a, and thus to be linearly polarized by the first polarizer 140a, subsequent to being emitted from the light-emitting element 120.

The second polarizer 140b may be disposed at a position through which light flowing into the photo-detective element 130 passes, and may be at least partially overlapped with the photo-detective element 130 in the thickness direction of the light-transmitting layer 110 (e.g., the Z direction). For example, the second polarizer 140b may be embedded in the light-transmitting layer 110. For example, the second polarizer 140b may be attached to the photo-detective element 130. For example, the second polarizer 140b may be disposed to be overlapped with the photo-detective element 130 on the light-transmitting layer 110 in a direction along which the photo-detective element 130 is configured to receive light (e.g., L4). For example, the photo-detective element 130 may be configured to detect (e.g., absorb and/or photoeletrically convert) light that is received at the photo-detective element 130 (e.g., incident on the photo-detective element) in a first direction (e.g., the Z direction), which may be the same direction (e.g., parallel to said direction) in which the light-emitting element 120 is configured to emit light (e.g., the light-emitting element 120 may be configured to emit light L1 in the -Z direction and the photo-detective element 130 may be configured to receive light L4 in the +Z direction). The second polarizer 140b may be positioned to at least partially overlap the photo-detecting element 130 in the in the first direction (e.g., the Z direction) that the photo-detecting element 130 is configured to receive light (e.g., L4), such that the biosignal sensor 100 is configured to cause the photo-detecting element 130 to receive light (e.g., L4) that passes through the second polarizer 140b (e.g., light L4 passing in the +Z direction). The direction along which the light-emitting element 120 is configured to emit light (e.g., the Z-direction) may be the same direction along which the first polarizer 140a at least partially overlaps the light-emitting element 120 and may also be the same direction along which the second polarizer 140b at least partially overlaps the photo-detective element 130. The second polarizer 140b may be configured to linearly polarize light (e.g., L4) flowing into the photo-detective element 130 in advance, such that the second polarizer 140b selectively transmits the linearly polarized light (e.g., L4), such that the biosignal sensor 100 is configured, based on the relative positioning of the photo-detective element 130 and the second polarizer 140b, to cause at least some, or all, of the light received at the photo-detective element 130 to pass through the second polarizer 140b, and thus to be linearly polarized by the second polarizer 140b, prior to being received at the photo-detective element 130.

As shown in FIG. 2, the first polarizer 140a completely overlaps the light-emitting element 120 in the Z direction, and the second polarizer 140b completely overlaps the photo-detective element 130 in the Z direction, such that the widths of the first and second polarizers 140a, 140b in the X direction are greater than the respective widths in the X direction of the light-emitting element 120 and the photo-detective element 130, but example embodiments are not limited thereto. For example, the first polarizer 140a may only partially overlap the light-emitting element 120 in the Z direction and/or the second polarizer 140b may only partially overlap the photo-detective element 130 in the Z direction.

As shown in FIG. 2, the first and second polarizers 140a and 140b may each extend in at least a first direction (e.g., the X direction and/or Y direction), and the first and second polarizers 140a and 140b may be adjacent to, and may be in contact with each other or isolated from direct contact with each other, in the same first direction (e.g., the X direction and/or the Y direction). Additionally, as shown in FIG. 2, the first and second polarizers 140 and 140b may each at least partially overlap with the light-emitting element 120 and the photo-detective element 130, respectively, in a second direction that is different from (e.g., perpendicular to) the first direction (e.g., the Z direction). The first and second polarizers 140a and 140b may be extend in a common plane in the X direction and Y direction, but example embodiments are not limited thereto, and in some example embodiments the first and second polarizers 140a and 140b may extend in parallel in separate planes in the X direction and Y direction that are offset in the Z direction.

It will be understood that an element that is "embedded" within another element may be located partially or entirely within a volume space defined by some or all outer surfaces of the other element, such that the element that is embedded within the other element may be partially or completely isolated from direct exposure to an exterior of the other element. For example, as shown in at least FIG. 2, the first and second polarizers 140a and 140b are each located within the volume space defined by the outer surfaces of the light-transmitting layer 110 (including upper surface 110S), such that the first and second polarizers 140a and 140b are each isolated from direct exposure to an exterior of the light-transmitting layer 110, and thus the first and second polarizers 140 and 140b may be understood to each be embedded within the light-transmitting layer 110.

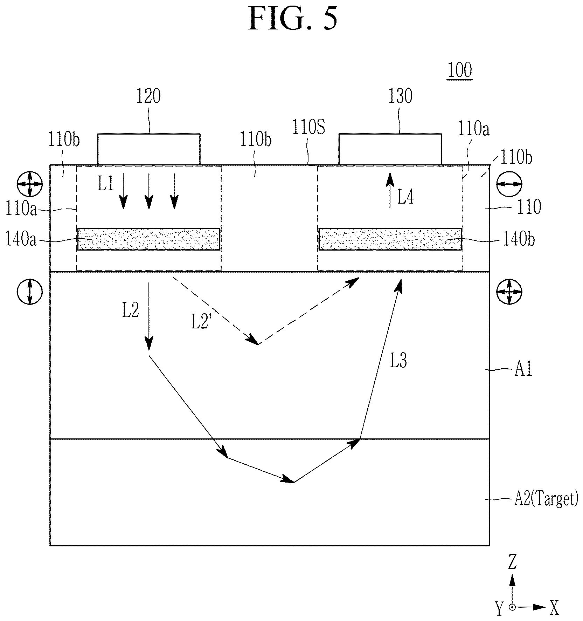

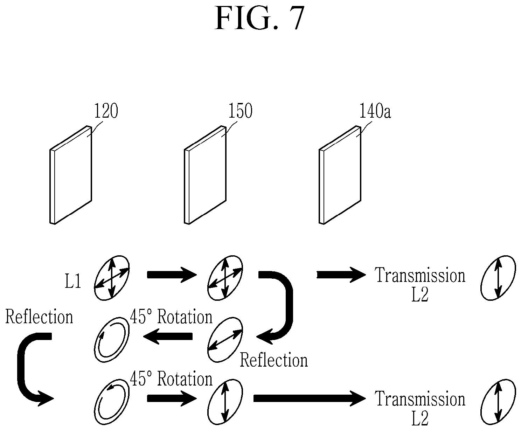

A polarization direction of the second polarizer 140b may be different from the polarization direction of the first polarizer 140a. As shown in at least FIGS. 5 and 7, the second polarizer 140b may be configured to selectively block light in a direction parallel to the linearly polarized light passing through the first polarizer 140a and may be configured to selectively transmit light in a different direction from the linearly polarized light passing through the first polarizer 140a.

For example, an angle between the polarization direction of the second polarizer 140b and the polarization direction of the first polarizer 140a may be, for example, greater than or equal to about 30 degrees, greater than or equal to 35 degrees, greater than or equal to 40 degrees, greater than or equal to 50 degrees, greater than or equal to about 55 degrees, or greater than or equal to about 60 and for example less than or equal to about 90 degrees, less than or equal to about 85 degrees, or less than or equal to about 80 degrees, for example about 30 degrees to about 90 degrees, about 40 degrees to about 90 degrees, about 50 degrees to about 90 degrees, about 55 degrees to about 90 degrees, or about 60 degrees to about 90 degrees. For example, the polarization direction of the second polarizer 140b may be perpendicular (e.g., substantially perpendicular) to the polarization direction of the first polarizer 140a.

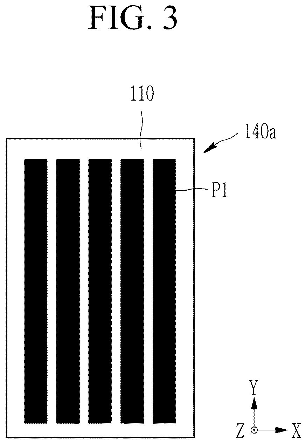

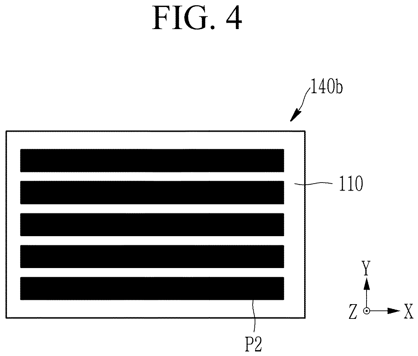

FIG. 3 is a plan view showing an example of a first polarizer and FIG. 4 is a plan view showing an example of a second polarizer, according to some example embodiments.

Referring to FIGS. 3 and 4, the first polarizer 140a and the second polarizer 140b may each be embedded in the light-transmitting layer 110 and each may include a plurality of patterns P1 and P2 extending in parallel in one direction at a particular (or, alternatively, predetermined) interval. For example, the first polarizer 140a may include a plurality of patterns P1, also referred to herein as a plurality of first patterns, extending in parallel in a first direction (e.g., Y direction) with a particular (or, alternatively, predetermined) interval, and the second polarizer 140b may include a plurality of patterns P2, also referred to herein as a plurality of second patterns, extending in parallel in a second direction (e.g., X direction) different from the first direction (e.g., perpendicular to the first direction as shown with regard to the Y direction and the X direction) with a particular (or, alternatively, predetermined) interval. The first direction may be a direction parallel to the polarization direction of the first polarizer 140a, and the second direction may be a direction parallel to the polarization direction of the second polarizer 140b. The angle between the first and second directions may be greater than or equal to about 30 degrees, greater than or equal to 35 degrees, greater than or equal to 40 degrees, greater than or equal to 50 degrees, greater than or equal to about 55 degrees, or greater than or equal to about 60 and for example less than or equal to about 90 degrees, less than or equal to about 85 degrees, or less than or equal to about 80 degrees, for example about 30 degrees to about 90 degrees, about 40 degrees to about 90 degrees, about 50 degrees to about 90 degrees, about 55 degrees to about 90 degrees, or about 60 degrees to about 90 degrees. For example, the first direction and the second direction may be perpendicular. For example, the first direction and the second direction may be substantially perpendicular. The plurality of patterns P1 and P2 may each be embedded within the light-transmitting layer 110.

The plurality of patterns P1 and P2 may be a grid polarizer made of (e.g., at least partially comprising) a metal, a dichroic dye, a polymer, or a combination thereof. Each pattern P1 and P2 may have, for example, a width of about 10 nm to about 500 nm, and within the range, about 20 nm to about 400 nm, about 30 nm to about 300 nm, about 30 nm to about 200 nm, or about 30 nm to about 100 nm, but is limited thereto. The interval between adjacent patterns P1 and P2 may be, for example, about 10 nm to about 500 nm, and within the above range, about 20 nm to about 400 nm, about 30 nm to about 300 nm, about 30 nm to about 200 nm, or about 30 nm to about 100 nm, but is not limited thereto.

FIG. 5 is a schematic view showing an example of a path of light in the biosignal sensor of FIGS. 1 and 2, according to some example embodiments.

Referring to FIG. 5, the light-emitting element 120 may be configured to emit unpolarized light L1, and when the unpolarized light L1 passes through the first polarizer 140a, light including one polarization orthogonal component out of two polarization orthogonal components, that is, a polarization component parallel to the polarization direction of the first polarizer 140a, may be transmitted, but light including the other polarization orthogonal component may be blocked. Accordingly, the light passing through the first polarizer 140a may be linearly polarized light in a parallel direction (e.g., Y direction) to the polarization direction of light the first polarizer 140a.

Most of the linearly polarized light L2 may reach the target A2 of a living body such as a vessel and thus be scattered and/or reflected. Herein, since the scattered and/or reflected light L3 may lose polarization of the linearly polarized light L2 and it is substantially unpolarized light with polarization components in all directions. In addition, since light scattered and/or reflected by a target such as blood vessels deep down from the skin is larger than light scattered by a target such as skin tissues not that deep from the skin surface, polarization information polarized from the first polarizer 140a may be more lost, and accordingly, the light scattered and/or reflected by the target such as blood vessels may more include the polarization component in a different direction from the polarization direction of the first polarizer 140a.

This scattered and/or reflected unpolarized light may be changed into linearly polarized light L4 parallel (e.g., an X direction) to the polarization direction of the second polarizer 140b, while it passes the second polarizer 140b, and thus the linearly polarized light L4 may flow into the photo-detective element 130. As described above, since the polarization direction of the second polarizer 140b differs from that of the first polarizer 140a, the linearly polarized light L4 flowing into the photo-detective element 130 may exclude light of the parallel direction (e.g., a Y direction) to the polarization direction of the first polarizer 140a but mainly include the scattered and/or reflected light by the target A2 such as blood vessel. Thus, the second polarizer 140b may be configured to selectively block light having a polarization in a polarization direction that is parallel to the polarization direction of the first polarizer 140a (e.g., the Y direction), and thus selectively block light having a polarization in a polarization direction that is parallel to a polarization direction of the linearly polarized light passing through the first polarizer 140a, and the second polarizer 140b may be configured to selectively transmit light having a polarization in a separate polarization direction that is different from (e.g., perpendicular to) the polarization direction of the linearly polarized light passing through the first polarizer (e.g., the X direction). Accordingly, a signal-to-noise ratio (STN) of the scattered and/or reflected light may be increased.

In some example embodiments, a portion L2' of the linearly polarized light passing through the first polarizer 140a may not reach the target A2 such as blood vessels but be lost in the light-transmitting layer 110 or directly reflected by the skin A1 toward the second polarizer 140b, but as described above, since the polarization direction of the second polarizer 140b differs from that of the first polarizer 140a, the portion L2' of the linearly polarized light may not pass the second polarizer 140b but be blocked. Accordingly, the second polarizer 140b may be configured to effectively block light not reaching the bio target such as blood vessels and thus having no bio-information and thereby, effectively reduce noise having no bio-information out of signals obtained from the photo-detective element 130.

When the biosignal sensor 100 is attached or placed close to the skin (e.g., lower surface 110L is in direct contact with skin A1) like a medical device-type sensor or a watch-type sensor, signal crosstalk may occur due to changes in the position and/or angle between the skin and the sensor due to skin motions. Herein, the changes in the position and/or angle between the skin and the sensor may occur due to the skin motions such as twisting, pulling, pressing, and/or the like. The signal crosstalk due to the changes in the position and/or angle between the skin and the sensor may deteriorate accuracy of the bio-information.

Specifically, as described above, a portion of the incident light toward the skin may be scattered and reflected by the skin, and another portion of the incident light may be scattered and reflected by the blood vessels. An amount of the light scattered and reflected by the skin is in general constant over time, but an amount of the light scattered and reflected by the blood vessels may be periodically or aperiodically changed over time by biosignal changes such as a blood flow due to contraction/relaxation of blood. The signals obtained from the scattered and/or reflected light may include a DC component scattered and/or reflected by the skin and an AC component showing the biosignal changes such as the blood flow, wherein when there are no skin motions, the constant DC component of the light scattered and/or reflected by the skin and the AC component showing the biosignal changes are clearly distinguished and have no crosstalk, but when there are skin motions such as twisting, pulling, pressing, and/or the like, the position and angle of the light-emitting element 120 and/or the photo-detective element 130 may be changed, and accordingly, the DC component of the light scattered and/or reflected by the skin may be largely changed and become unstable and thus be misunderstood with the AC component showing the biosignal changes such as a blood flow and work as noise and resultantly, deteriorate accuracy of the obtained bio-information.

Some example embodiments, as described above, may make a particular (or, alternatively, predetermined) polarization component out of the light emitted from the light-emitting element 120 incident into the skin and then, make other polarization components except for the particular (or, alternatively, predetermined) polarization component selectively transmit the photo-detective element 130 to suppress or prevent the unstable DC component due to skin scattering changes by the skin motions such as twisting, pulling, pressing, and/or the like from flowing into the photo-detective element 130 and thus reduce the noise. Accordingly, efficiency of the biosignal sensor 100 and the accuracy of the bio-information may be increased by reducing the noise due to the position and/or angle changes between the skin and the sensor and effectively increasing signals of the light scattered and reflected by the target such as the blood vessels.

In some example embodiments, the light-emitting element 120 may be omitted from the biosignal sensor 100, and the biosignal sensor 100 may be configured to utilize incident light L1 which may be received from an ambient environment and/or a separate light source that is separate from the biosignal sensor (e.g., a separate light emitting diode (LED).

Hereinafter, another example of a biosignal sensor according to some example embodiments is described.

FIG. 6 is a cross-sectional view of another example of the biosignal sensor of FIG. 1 taken along line II-II' in FIG. 1, according to some example embodiments, and FIG. 7 is a schematic view showing a change in polarization of the biosignal sensor of FIG. 6, according to some example embodiments.

Referring to FIG. 6, a biosignal sensor 100 according to some example embodiments includes a light-transmitting layer 110; a light-emitting element 120; a photo-detective element 130; and a polarizer 140 including a first polarizer 140a and a second polarizer 140b as some example embodiments, including the example embodiments shown in FIGS. 1-5. In some example embodiments, the light-emitting element 120 may be omitted from the biosignal sensor 100 shown in FIG. 6, and the biosignal sensor 100 may be configured to utilize incident light L1 which may be received from an ambient environment and/or a separate light source that is separate from the biosignal sensor (e.g., a separate light emitting diode (LED).

However, unlike some example embodiments, including the example embodiments shown in FIGS. 1-5, the biosignal sensor 100 according to some example embodiments, including the example embodiments shown in FIGS. 6-7, further includes a phase retarder 150. The phase retarder 150 may be disposed at a position through which light emitted from the light-emitting element 120 passes. For example, the phase retarder 150 may be attached to the light-emitting element 120. For example, the phase retarder 150 may be positioned to be at least partially overlapped with the light-emitting element 120 in the thickness direction (e.g., the Z direction) inside the light-transmitting layer 110. For example, the phase retarder 150 may extend in same first direction in which the first polarizer 140a extends (e.g., the X direction and/or the Y direction) and thus may extend in parallel with the first polarizer 140a and may further at least partially overlap with the first polarizer 140a in a second direction different from the first direction (e.g., the Z direction as shown in FIG. 6). The phase retarder may at least partially overlap both the first polarizer 140a and the light-emitting element 120 in the Z direction. For example, the phase retarder 150 may be disposed between the light-emitting element 120 and the first polarizer 140a (e.g., in the thickness direction, e.g., the Z direction). The phase retarder 150 may include, for example, a polymer such as polycarbonate, polyimide, cycloolefin, cellulose ester, or a combination thereof. The phase retarder 150 may include, for example, a birefringent material such as liquid crystals. The phase retarder 150 may be, for example, a λ/4 phase retarder.

Referring to FIG. 7, the phase retarder 150 may change the polarization direction of light (e.g., left-handed circularly polarized light) not passing the first polarizer 140a but reflected by it and then, change again the polarization direction of the light reflected by the light-emitting element 120 and thus convert it into the linearly polarized light L2 in a parallel direction to the polarization direction of the first polarizer 140a, and this linearly polarized light L2 may pass through the first polarizer 140a. Accordingly, the light amount of the linearly polarized light L2 passing through the first polarizer 140a out of the light L1 emitted from the light-emitting element 120 may be increased to enhance light conversion efficiency. For example, a ratio of the linearly polarized light L2 to the light L1 emitted from the light-emitting element 120 may be, for example, greater than or equal to about 60%, greater than or equal to about 65%, greater than or equal to about 70%, or greater than or equal to about 75%.

Another example of a biosignal sensor according to some example embodiments is described below.

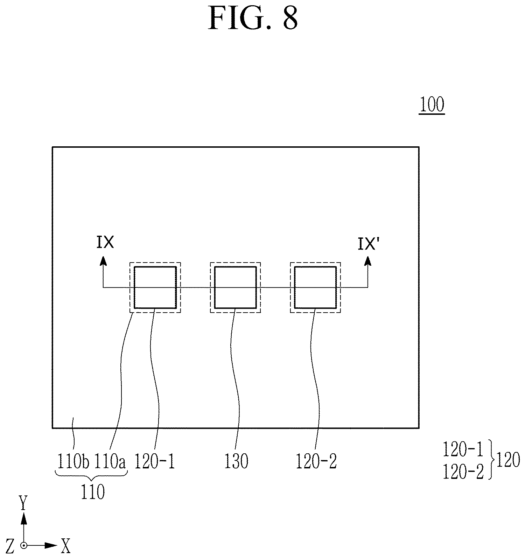

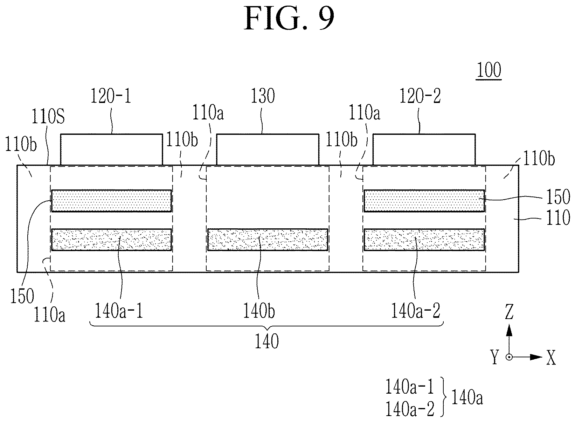

FIG. 8 is a plan view showing another example of a biosignal sensor according to some example embodiments, and FIG. 9 is a cross-sectional view of the biosignal sensor of FIG. 8 taken along line IX-IX' in FIG. 8, according to some example embodiments.

Referring to FIGS. 8 and 9, the biosignal sensor 100 according to some example embodiments includes a light-transmitting layer 110; a light-emitting element 120; a photo-detective element 130; a polarizer 140 including a first polarizer 140a and a second polarizer 140b; and optionally a phase retarder 150, as in some example embodiments, including the example embodiments shown in FIGS. 1-7. Detailed description is the same as described above.

However, the biosignal sensor 100 according to some example embodiments, including the example embodiments shown in FIGS. 8-9, includes a plurality of light-emitting elements 120-1 and 120-2, unlike some example embodiments, including the example embodiments shown in FIGS. 1-7. The light-emitting element 120 includes a first light-emitting element 120-1 and a second light-emitting element 120-2 configured to emit light in different wavelength spectra. The first light-emitting element 120-1 and the second light-emitting element 120-2 may be used to detect objects having different absorption and/or reflection characteristics. For example, the first light-emitting element 120-1 may be configured to emit light in a first wavelength spectrum, and the second light-emitting element 120-2 may be configured to emit light in a second wavelength spectrum that is a longer wavelength spectrum than the first wavelength spectrum. For example, the first light-emitting element 120-1 may be a green light-emitting element configured to emit light in a green wavelength spectrum, and the second light-emitting element 120-2 may be a red light-emitting element configured to emit light in a red wavelength spectrum or an infrared light-emitting element configured to emit light in an infrared wavelength spectrum. The green light-emitting element and the red/infrared ray light-emitting element may be used for absorption and/or reflection properties of, for example, oxyhemoglobin (HbO₂) and hemoglobin (Hb) in blood vessels. As shown in FIG. 8, the first polarizer 140a may include a third polarizer 140a-1 that is overlapped with the first light-emitting element 120-1 in the thickness direction (e.g., Z direction) and a fourth polarizer that is overlapped with the second light-emitting element 120-2 in the thickness direction (e.g., Z direction). A polarization direction of the third polarizer 140a-1 and a polarization direction of the fourth polarizer 140a-2 may be in parallel with each other. In some example embodiments, the polarization direction of the third polarizer 140a-1 and the polarization direction of the fourth polarizer 140a-2 may each be perpendicular to the polarization direction of the second polarizer 140b.

In some example embodiments, the light-emitting elements 120-1 and 120-2 may be omitted from the biosignal sensor 100 shown in FIGS. 8-9, and the biosignal sensor 100 may be configured to utilize incident light L1 which may be received from an ambient environment and/or a separate one or more light sources that are separate from the biosignal sensor (e.g., a separate light emitting diode (LED), such that the third and fourth polarizers 140a-1 and 140a-2 are configured to receive and linearly polarize incident light received from one or more, or separate, respective external light sources.

The aforementioned biosignal sensor 100 may be applied in the form of an array arranged along rows and/or columns.

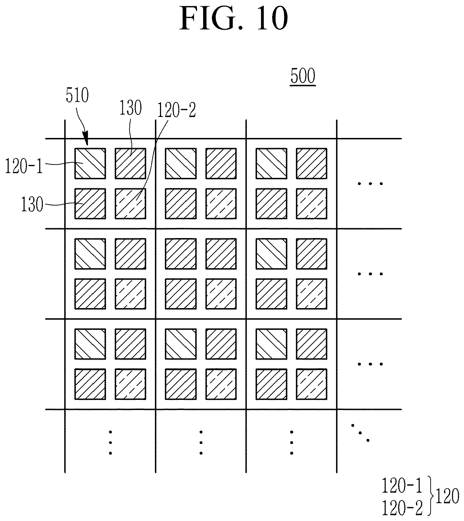

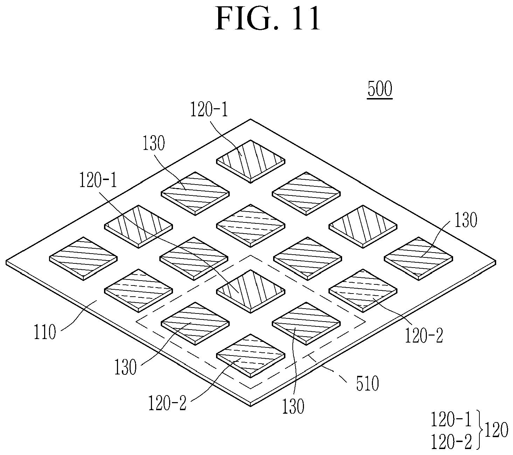

FIG. 10 is a schematic view showing an example arrangement of a biosignal sensor array according to some example embodiments and FIG. 11 is a schematic view showing a portion of the biosignal sensor array of FIG. 10, according to some example embodiments.

Referring to FIG. 10, a biosignal sensor array 500 may have a matrix arrangement in which a plurality of unit element groups 510 are repeatedly arranged along rows and/or columns. The arrangement of the unit element group 510 may be, for example, a Bayer matrix, a PenTile matrix, and/or a diamond matrix, but is not limited thereto.

In the drawing, all unit element groups 510 are shown to have the same size but not limited thereto, and at least one unit element group 510 of the unit element groups 510 may be larger or smaller than the other unit element groups 510. In the drawing, all the unit element groups 510 are shown to have the same shape but not limited thereto, and at least one unit element group 510 of the unit element groups 510 may have a different shape from those of the other unit element groups 510.

Each unit element group 510 may be arranged on the above-described light-transmitting layer 110 and may include the light-emitting element 120 and photo-detective element 130, and even if not shown, the aforementioned first polarizer 140a disposed to be overlapped with the light-emitting element 120, the aforementioned second polarizer 140b disposed to be overlapped with the photo-detective element 130, and optionally a phase retarder 150 between the light-emitting element 120 and the first polarizer 140a. In some example embodiments, each unit element group 510, including the unit element groups shown in FIGS. 11-12, includes at least one light-emitting element 120 (e.g., light-emitting element 120-1 and/or light-emitting element 120-2), at least one photo-detective element 130, at least one first polarizer 140a, and at least one second polarizer 140b. FIGS. 10 and 11 show that each unit element group 510 includes one first light-emitting element 120-1, one second light-emitting element 120-2, and two photo-detective elements 130 but is not limited thereto and may include at least one of the first or second light-emitting elements 120-1 or 120-2 and at least one photo-detective element 130. Either one of the first light-emitting element 120-1 or the second light-emitting element 120-2 may be omitted.

The light-emitting element 120 and the photo-detective element 130 included in each unit element group 510 may have a size (dimension) of several to hundreds of micrometers. For example, the light-emitting element 120 and the photo-detective element 130 included in each unit element group 510 may independently have a width, a length, and a thickness of greater than or equal to about 1 µm and less than about 1000 µm and within the range, about 10 µm to about 800 µm, about 10 µm to about 700 µm, about 10 µm to about 600 µm, or about 10 µm to about 500 µm.

This biosignal sensor array 500 includes the plurality of light-emitting elements 120 and the photo-detective elements 130 arranged along rows and/or columns and thus may more easily detect biological signals. Accordingly, it will be understood that the biosignal sensor array 500 may include a biosignal sensor according to some example embodiments (e.g., biosignal sensor 100, and/or at least a portion of each unit element group 510, including a portion of the unit element group 510 that excludes or includes the pressure sensor 300, may correspond to a biosignal sensor 100)

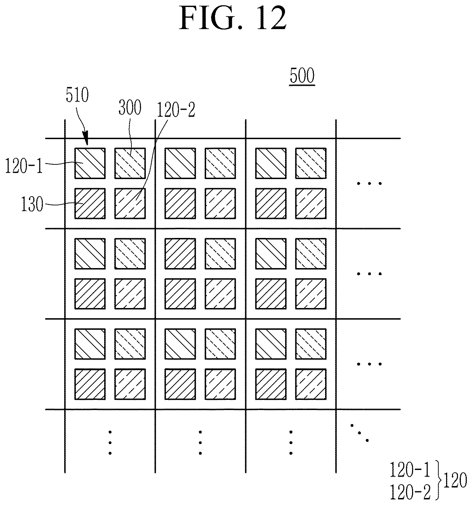

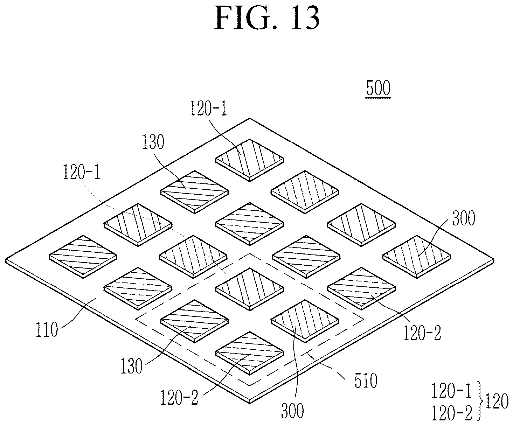

FIG. 12 is a schematic view showing an arrangement of another example of a biosignal sensor array according to some example embodiments and FIG. 13 is a schematic view showing a portion of the biosignal sensor array of FIG. 12, according to some example embodiments.

The biosignal sensor array 500 according to some example embodiments has a matrix arrangement in which a plurality of unit element groups 510 are repeatedly arranged along rows and/or columns, as in some example embodiments, including the example embodiments shown in FIG. 11, and each unit element group 510 includes a light-emitting element 120 and a photo-detective element 130, and even if not shown, the aforementioned polarizer 140 including the first polarizer 140a and second polarizer 140b, and optionally the phase retarder 150.

However, the biosignal sensor array 500 according to some example embodiments, including the example embodiments shown in FIG. 12, further includes a pressure sensor 300 in each unit element group 510, unlike some example embodiments, including the example embodiments shown in FIG. 11. That is, each unit element group 510 includes a light-emitting element 120, a photo-detective element 130, and a pressure sensor 300. FIGS. 12 and 13 for example show that each unit element group 510 includes one first light-emitting element 120-1, one second light-emitting element 120-2, one photo-detective element 130, and one pressure sensor 300 but is not limited thereto and may include at least one first and/or second light-emitting element 120-1 and/or 120-2, at least one photo-detective element 130, and at least one pressure sensor 300. Either one of the first light-emitting element 120-1 or the second light-emitting element 120-2 may be omitted. In some example embodiments, each unit element group 510 may be understood to be a separate biosignal sensor.

The pressure sensor 300 is a sensor configured to detect pressure changes. Accordingly, the pressure sensor 300 among a plurality of pressure sensors 300 arranged in the biosignal sensor array 500 may be used to specify where a pressure occurs, and thus the corresponding unit element group 510 alone may be selectively operated to effectively detect biological signals at the specific position of a target such as a blood vessel. The pressure sensor 300 may include a pair of electrodes and a pressure sensitive layer between electrodes, and the pressure sensitive layer may include, for example, carbon nanotubes, metal nanowires, metal nanoparticles, a dielectric material, conductive polymer, or a combination thereof.

For example, a method of operating the biosignal sensor array 500 according to some example embodiments may include, for example, specifying where the pressure sensor 300 detecting the pressure is among the plurality of unit element groups 510 of the biosignal sensor array 500 and selectively operating a unit element group 510 to which the pressure sensor 300 belongs. The selective operation of the unit element group 510 to which the pressure sensor 300 detecting the pressure belongs may include, for example, radiating light into the light-emitting element 120 of the unit element group 510 to which the pressure sensor 300 sensing the pressure belongs and then, absorbing the light reflected by the target, for example, a blood vessel in the photo-detective element 130 and converting the absorbing light into electrical signals.

The aforementioned biosignal sensor 100 or biosignal sensor array 500 according to any example embodiments may be applied to (e.g., included in) various sensor systems configured to collect biosignal information, and may be used to obtain biosignals temporarily or in real time. The biosignal sensor 100 may be applied to, for example, a wearable bioelectronic device or a skin attachable device that is directly attached to the skin, and may be used to obtain a physiological signal such as blood flow temporarily or in real time, but is not limited thereto. The sensor system may be, for example, a patch-type biosignal sensor system or a band-type biosignal sensor system.

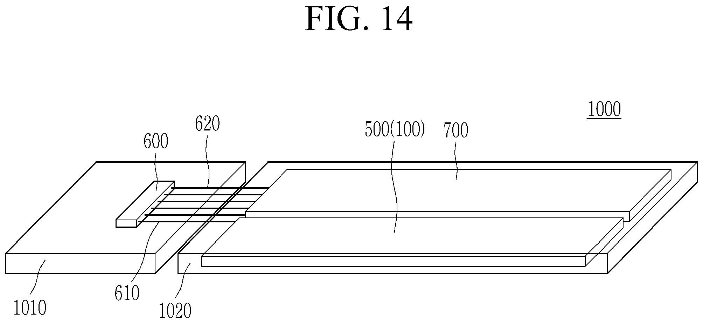

FIG. 14 is a schematic view showing an example of a sensor system according to some example embodiments.

Referring to FIG. 14, a sensor system 1000 according to some example embodiments may be a patch- or band-type biosignal sensor system, and may include the aforementioned biosignal sensor 100 or biosignal sensor array 500; IC and/or a processor 600 configured to process biosignals obtained from the biosignal sensor 100 or biosignal sensor array 500, and a display region 700 configured to display the obtained biological signals into various letters and/or images.

As shown, the IC and/or processor 600 may be electrically coupled to the aforementioned biosignal sensor 100 or biosignal sensor array 500 and the display region 700 via separate, respective conductive lines 610, 620. In some example embodiments, the conductive lines 610, 620 may be conductive wires, conductive traces, an electrical bus, any combination thereof, or the like.

As shown, the IC and/or processor 600 may be on or at least partially embedded in a substrate 1010. The biosignal sensor 100 or biosignal sensor array 500, and the display region 700 may be on or at least partially embedded in a substrate 1020. In some example embodiments, substrate 1010 and 1020 may be separate portions of a single, continuous substrate that is a single piece of material. In some example embodiments, the substrate 1010 and 1020 may each at least partially comprise a same material as a material included in at least a portion of the light-transmitting layer 110 as described herein. In some example embodiments at least the substrate 1020 comprises the light-transmitting layer, such that the substrate 1020 is a single piece of material having a portion thereof that is the light-transmitting layer 110 of a biosignal sensor 100 or biosignal sensor array 500.

The display region 700 may be a display panel, including an LED display panel, an OLED display panel, a liquid crystal display (LCD) panel, any combination thereof, or the like. The IC and/or processor 600 may be configured to display information on the display region 700 based on information obtains from the biosignal sensor 100 or biosignal sensor array 500.

The IC and/or processor 600 may include, may be included in, and/or may be implemented by one or more instances of processing circuitry such as hardware including logic circuits; a hardware/software combination such as a processor executing software; or a combination thereof. For example, the processing circuity more specifically may include, but is not limited to, a central processing unit (CPU), an arithmetic logic unit (ALU), a graphics processing unit (GPU), an application processor (AP), a digital signal processor (DSP), a microcomputer, a field programmable gate array (FPGA), and programmable logic unit, a microprocessor, application-specific integrated circuit (ASIC), a neural network processing unit (NPU), an Electronic Control Unit (ECU), an Image Signal Processor (ISP), and the like. In some example embodiments, the processing circuitry may include a non-transitory computer readable storage device (e.g., memory), for example a solid state drive (SSD), storing a program of instructions, and a processor configured to execute the program of instructions to implement the functionality and/or methods performed by some or all of the IC and/or processor 600 and thus the functionality and/or methods performed by some or all of the sensor system 1000.

As an example, the biosignal sensor and/or biosignal sensor array according to some example embodiments (e.g., biosignal sensor 100 and/or biosignal sensor array 500) may be a photoplethysmography (PPG) sensor device, a functional near infrared spectroscopy (fNIRS) for brain imaging, or the like, but example embodiments are not limited thereto.

Hereinafter, some example embodiments are illustrated in more detail with reference to examples.

### Optical Simulation

Noise change according to skin motions of a biosignal sensor is evaluated.

The evaluation of noise change is performed by using a LightTools software. In the biosignal sensor shown in FIGS. 1 and 2, when the light-emitting element emits light and the photo-detective element detects scattered and/or reflected by a living body (skin, blood vessels, and the like), noise changes according to skin motions depending on the presence or absence of a polarizer are evaluated.

The structure of the biosignal sensor is set as follows.
- Biosignal sensor: photoplethysmography (PPG) sensor
- Emission spectrum of light-emitting element: 550 nm to 650 nm (λₘₐₓ= 600 nm)
- Assuming that internal quantum efficiency of the photo-detective element is 100%.
- Interval between the light-emitting element and the photo-detective element: 0 to 1 mm
- Light-transmitting layer: stretchable light-transmitting layer
- Thickness of light-transmitting layer: 0.05 mm
- Skin composition: skin thickness of 1.5 mm, blood vessel thickness of 1 mm, fat thickness of 2 mm, and muscle thickness of 30 mm
- The polarization direction of the first polarizer and the polarization direction of the second polarizer are perpendicular (90 degrees)

### (1) Change in noise according to the angle change between skin- and photo-detective element

When the photo-detective element is ±10 ° tilted against the plane direction of the light-transmitting layer due to the skin motion (twisting), the noise changes depending on the presence or absence of a polarizer are evaluated.

The results are shown in Table 1.

**Table 1**

| | Signal-to-motion noise (signal: 1) |
|---|---|
| Example 1 (with polarizer) | 2.5 |
| Comparative Example 1 (without polarizer) | 4.8 |

### (2) Noise changes according to the interval between the light-emitting element and the photo-detective element

When the photo-detective element is about ±20 ° tilted against the plane direction of the light-transmitting layer due to the skin motion (pulling), the noise changes depending on the presence or absence of a polarizer are evaluated.

The results are shown in Table 2.

**Table 2**

| | Signal-to-motion noise (signal: 1) |
|---|---|
| Example 2 (with polarizer) | 4.1 |
| Comparative Example 2 (without polarizer) | 5.0 |

### (3) Noise changes according to uneven thickness of the light-transmitting layer

When a thickness of the stretchable light-transmitting layer is changed by the skin motion (pressing) (when the thickness is not uniform, ± 0.15 nm from an average thickness), the noise changes depending on the presence or absence of a polarizer are evaluated.

The results are shown in Table 3.

**Table 3**

| | Signal-to-motion noise (signal: 1) |
|---|---|
| Example 3 (with polarizer) | 0.75 |
| Comparative Example 3 (without polarizer) | 1.5 |

Referring to Tables 1 to 3, the biosignal sensor including the polarizer turns out to greatly reduce the noises according to the skin motions, compared with the biosignal sensor including no polarizer.

### EXAMPLE I

### Example 4

A polarizing film (Dichroic film polarizer for visible light, Thorlabs, Inc.) is attached respectively to a green light-emitting element (BioMon Sensor, Model Name: SFH7060, OSRAM SYLVANIA Inc.), a red light-emitting element (BioMon Sensor, Model Name: SFH7060, OSRAM SYLVANIA Inc.), and a photo-detective element (BioMon Sensor, Model Name: SFH7060, OSRAM SYLVANIA Inc.), and the obtained green light-emitting element, the red light-emitting element, and the photo-detective element are disposed on a SEBS substrate at 4 mm intervals to manufacture a biosignal sensor. Herein, the polarizing film attached to the green light-emitting element is disposed in a perpendicular polarization direction with the polarizing film attached to the photo-detective element, and the polarizing film attached to the red light-emitting element is disposed in a parallel polarization direction with the polarizing film attached to the photo-detective element.

### Example 5

A biosignal sensor is manufactured according to the same method as Example 4 except that the polarizing film attached to the green light-emitting element is disposed in a parallel polarization direction with the polarizing film attached to the photo-detective element, and the polarizing film attached to the red light-emitting element is disposed in a perpendicular polarization direction with the polarizing film attached to the photo-detective element.

### Evaluation I

Performances of the biosignal sensors according to Examples 4 and 5 are evaluated.

The performances of the biosignal sensors are evaluated by attaching the biosignal sensors around the radial artery of the wrist and detecting signal (PPG signal) changes thereof.

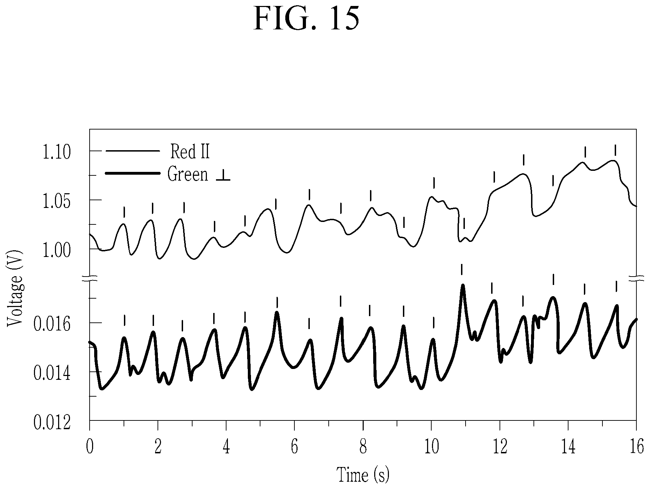

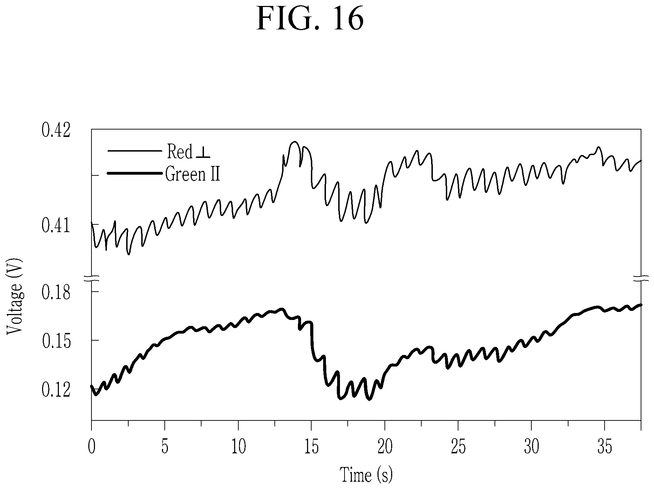

The results are shown in FIGS. 15 and 16.

FIG. 15 is a graph showing biosignal changes of a biosignal sensor according to Example 4, and FIG. 16 is a graph showing biosignal changes of the biosignal sensor according to Example 5.

Referring to FIG. 15, in the biosignal sensor of Example 4, light signals in the green wavelength spectrum receive relatively less influences from the motions, but light signals in the red wavelength spectrum receive relatively more influences from the motions.

Referring to FIG. 16, in the biosignal sensor of Example 5, light signals in the red wavelength spectrum receive relatively less influences from the motions, but light signals in the green wavelength spectrum receive relatively more influences from the motions.

Accordingly, it is confirmed that when the polarization directions of the polarizing film attached to the light-emitting element and the polarizing film attached to the photo-detective element are parallel each other, noises due to the motions are relatively high, but when the polarization directions of the polarizing film attached to the light-emitting element and the polarizing film attached to the photo-detective element are perpendicular each other, the noises due to the motions are reduced.

### EXAMPLE II

### Example 6

A biosignal sensor is manufactured by disposing a red light-emitting element (an area: 0.5 x 0.5 mm²) and a photo-detective element (an area: 0.5 x 0.5 mm²) at intervals of 0.8 mm on a SEBS substrate, attaching each polarizing film (Dichroic film polarizer for visible light, Thorlabs, Inc.) (each area: 0.5 x 0.5 mm²) to the bottom of the SEBS substrate at the corresponding positions to the red light-emitting element and the photo-detective element. The red light-emitting element is formed by sequentially stacking a lower electrode (IZO) / a hole auxiliary layer / an organic light emitting layer / an electron auxiliary layer / an upper electrode (Al), and the photo-detective element is formed by stacking a lower electrode (IZO) / a hole auxiliary layer/light-absorbing layer (SubNc/C60) / an electron auxiliary layer / an upper electrode (Al). The polarizing film attached to the red light-emitting element is disposed in a perpendicular polarization direction with the polarizing film attached to the photo-detective element.

### Comparative Example 4

A biosignal sensor is manufactured according to the same method as Example 6 except that the polarizing films are not included.

### Evaluation II

Performances of the biosignal sensors according to Example 6 and Comparative Example 4 are evaluated.

The performances of the biosignal sensors are evaluated by attaching the biosignal sensors to a finger or a wrist and detecting changes of signals (PPG signals) when to work out (exercise) and when to take a rest.

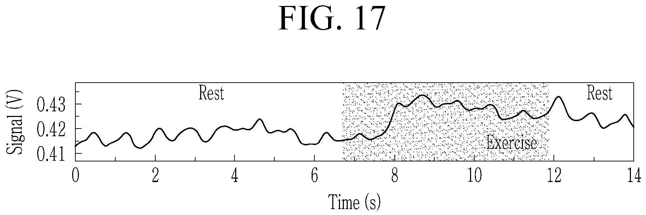

The results are shown in FIGS. 17 to and 18.

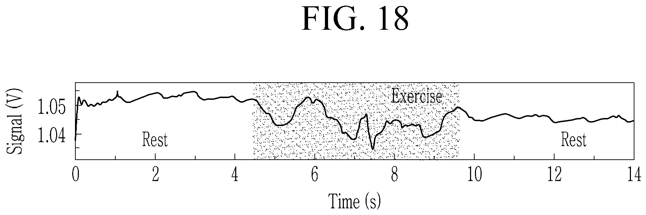

FIG. 17 is a graph showing biosignal changes of a biosignal sensor according to Example 4 and FIG. 18 is a graph showing biosignal changes of a biosignal sensor according to Comparative Example 4.

Referring to FIGS. 17 and 18, the biosignal sensors have different influences from the motions depending on the presence of the polarizer, and specifically, the biosignal sensor including the polarizer exhibited relatively less noise than the biosignal sensor not including the polarizer.

## Claims

1. A biosignal sensor (100), comprising:
a stretchable light-transmitting layer (110),
a light source (120) on the stretchable light-transmitting layer,
a light sensor (130) on the stretchable light-transmitting layer,
a first polarizer (140a) embedded within the stretchable light-transmitting layer, the first polarizer being configured to linearly polarize light emitted from the light source, such that the biosignal sensor is configured to cause at least some of the light emitted from the light source to be linearly polarized by the first polarizer subsequent to being emitted from the light source, and
a second polarizer (140b) embedded within the stretchable light-transmitting layer, the second polarizer being configured to linearly polarize light received at the light sensor, such that the biosignal sensor is configured to cause at least some of the light received at the light sensor to be linearly polarized by the second polarizer prior to being received at the light sensor,
wherein the stretchable light-transmitting layer includes a plurality of first regions (110a) and a second region (110b) between adjacent first regions of the plurality of first regions, the plurality of first regions each having a first elastic modulus, the second region having a second elastic modulus that is lower than the first elastic modulus,
the light source and the light sensor are each on a separate first region of the plurality of first regions, and
the second region is entirely continuously connected.

2. The biosignal sensor (100) of claim 1, wherein
the first polarizer (140a) at least partially overlaps with the light source in a first direction along which the light source is configured to emit light, and
the second polarizer (140b) at least partially overlaps with the light sensor in the first direction.

3. The biosignal sensor (100) of any preceding claim, wherein a polarization direction of the second polarizer (140b) is different from a polarization direction of the first polarizer (140a), and optionally wherein an angle between the polarization direction of the second polarizer and the polarization direction of the first polarizer is 30 degrees to 90 degrees.

4. The biosignal sensor (100) of claim 3, wherein the polarization direction of the second polarizer (140b) is perpendicular to the polarization direction of the first polarizer (140a).

5. The biosignal sensor (100) of any preceding claim, wherein the second polarizer (140b) is configured to selectively block light in a polarization direction parallel to a polarization direction of the linearly polarized light passing through the first polarizer (140a), and selectively transmit light in a separate polarization direction different from the polarization direction of the linearly polarized light passing through the first polarizer.

6. The biosignal sensor (100) of any preceding claim, wherein
the first polarizer (140a) includes a plurality of first patterns extending in a first direction, and
the second polarizer (140b) includes a plurality of second patterns extending in a second direction, the second direction being different from the first direction,
and optionally wherein the first direction and the second direction are perpendicular to each other.

7. The biosignal sensor (100) of claim 6,
wherein the plurality of first patterns extending in the first direction and the plurality of second patterns extending in the second direction are embedded within the light-transmitting layer.

8. The biosignal sensor of any preceding claim, further comprising:
a phase retarder between the light source and the first polarizer.

9. The biosignal sensor (100) of any preceding claim, wherein the biosignal sensor is a skin-attachable photoplethysmography, PPG, sensor.

10. A sensor system comprising the biosignal sensor (100) of any preceding claim.

11. A sensor array (500) comprising the biosignal sensor (100) of any of claims 1 to 9.

12. The sensor array (500) of claim 11, wherein
the sensor array includes a plurality of unit element groups (510), and
each unit element group of the plurality of unit element groups includes a biosensor according to any of claims 1-9, and optionally wherein the each unit element group further includes a pressure sensor (300).

13. The sensor array (500) of claim 12, wherein
the light source (120) includes a first light source (120-1) configured to emit light in a first wavelength spectrum and a second light source (120-2) configured to emit light in a second wavelength spectrum, the second wavelength spectrum being longer than the first wavelength spectrum,
the first polarizer (140a) includes
a third polarizer (140a-1) overlapped with the first light source, and
a fourth polarizer (140a-2) overlapped with the second light source, and
a polarization direction of the third polarizer and a polarization direction of the fourth polarizer are in parallel, and optionally wherein the polarization direction of the third polarizer and the polarization direction of the fourth polarizer are perpendicular to the polarization direction of the second polarizer (140b).

## Patentansprüche

1. Biosignalsensor (100), umfassend:
eine dehnbare lichtdurchlässige Schicht (110),
eine Lichtquelle (120) auf der dehnbaren lichtdurchlässigen Schicht,
einen Lichtsensor (130) auf der dehnbaren lichtdurchlässigen Schicht,
einen ersten Polarisator (140a), der in die dehnbare lichtdurchlässige Schicht eingebettet ist, wobei der erste Polarisator dazu konfiguriert ist, von der Lichtquelle emittiertes Licht linear zu polarisieren, sodass der Biosignalsensor dazu konfiguriert ist, zu bewirken, dass mindestens ein Teil des von der Lichtquelle emittierten Lichts, nachdem es von dem Lichtquelle emittiert wird, durch den ersten Polarisator linear polarisiert wird, und
einen zweiten Polarisator (140b), der in die dehnbare lichtdurchlässige Schicht eingebettet ist, wobei der zweite Polarisator dazu konfiguriert ist, an dem Lichtsensor empfangenes Licht linear zu polarisieren, sodass der Biosignalsensor dazu konfiguriert ist, zu bewirken, dass mindestens ein Teil des an dem Lichtsensor empfangenen Lichts, bevor es an dem Lichtsensor empfangen wird, durch den zweiten Polarisator linear polarisiert wird,
wobei die dehnbare lichtdurchlässige Schicht eine Vielzahl von ersten Bereichen (110a) und einen zweiten Bereich (1 10b) zwischen benachbarten ersten Bereichen der Vielzahl von ersten Bereichen enthält, wobei die Vielzahl von ersten Bereichen jeweils einen ersten Elastizitätsmodul aufweist, der zweite Bereich einen zweiten Elastizitätsmodul aufweist, der niedriger als der erste Elastizitätsmodul ist,
die Lichtquelle und der Lichtsensor jeweils in einem separaten ersten Bereich der Vielzahl von ersten Bereichen liegen, und
der zweite Bereich vollständig durchgehend verbunden ist.

2. Biosignalsensor (100) nach Anspruch 1, wobei
der erste Polarisator (140a) sich mindestens teilweise mit der Lichtquelle in einer ersten Richtung, entlang der die Lichtquelle zum Emittieren von Licht konfiguriert ist, überlappt und
der zweite Polarisator (140b) sich mindestens teilweise mit dem Lichtsensor in der ersten Richtung überlappt.

3. Biosignalsensor (100) nach einem vorhergehenden Anspruch, wobei sich eine Polarisationsrichtung des zweiten Polarisators (140b) von einer Polarisationsrichtung des ersten Polarisators (140a) unterscheidet und wobei optional ein Winkel zwischen der Polarisationsrichtung des zweiten Polarisators und der Polarisationsrichtung des ersten Polarisators 30 Grad bis 90 Grad beträgt.

4. Biosignalsensor (100) nach Anspruch 3, wobei die Polarisationsrichtung des zweiten Polarisators (140b) senkrecht zu der Polarisationsrichtung des ersten Polarisators (140a) ist.

5. Biosignalsensor (100) nach einem vorhergehenden Anspruch, wobei der zweite Polarisator (140b) dazu konfiguriert ist, selektiv Licht in einer Polarisationsrichtung zu blockieren, die parallel zu einer Polarisationsrichtung des linear polarisierten Lichts ist, das durch den ersten Polarisator (140a) hindurchgeht, und selektiv Licht in einer separaten Polarisationsrichtung durchzulassen, die sich von der Polarisationsrichtung des linear polarisierten Lichts unterscheidet, das durch den ersten Polarisator hindurchgeht.

6. Biosignalsensor (100) nach einem vorhergehenden Anspruch, wobei
der erste Polarisator (140a) eine Vielzahl von ersten Mustern, die sich in eine erste Richtung erstrecken, enthält und
der zweite Polarisator (140b) eine Vielzahl von zweiten Mustern, die sich in eine zweite Richtung erstrecken, enthält, wobei sich die zweite Richtung von der ersten Richtung unterscheidet,
und wobei optional die erste Richtung und die zweite Richtung senkrecht zueinander sind.

7. Biosignalsensor (100) nach Anspruch 6,
wobei die Vielzahl von ersten Mustern, die sich in die erste Richtung erstrecken, und die Vielzahl von zweiten Mustern, die sich in die zweite Richtung erstrecken, in die lichtdurchlässige Schicht eingebettet sind.

8. Biosignalsensor nach einem vorhergehenden Anspruch, ferner umfassend:
einen Phasenverzögerer zwischen der Lichtquelle und dem ersten Polarisator.

9. Biosignalsensor (100) nach einem vorhergehenden Anspruch, wobei der Biosignalsensor ein an der Haut anbringbarer Photoplethysmographie-Sensor, PPG-Sensor, ist.

10. Sensorsystem, den Biosignalsensor (100) nach einem vorhergehenden Anspruch umfassend.

11. Sensorarray (500), den Biosignalsensor (100) nach einem der Ansprüche 1 bis 9 umfassend.

12. Sensorarray (500) nach Anspruch 11, wobei
das Sensorarray eine Vielzahl von Einheitselementgruppen (510) enthält und jede Einheitselementgruppe der Vielzahl von Einheitselementgruppen einen Biosensor gemäß einem der Ansprüche 1-9 enthält,
und wobei optional jede Einheitselementgruppe ferner einen Drucksensor (300) enthält.

13. Sensorarray (500) nach Anspruch 12, wobei
die Lichtquelle (120) eine erste Lichtquelle (120-1), die dazu konfiguriert ist, Licht in einem ersten Wellenlängenspektrum zu emittieren, und eine zweite Lichtquelle (120-2), die dazu konfiguriert ist, Licht in einem zweiten Wellenlängenspektrum zu emittieren, enthält, wobei das zweite Wellenlängenspektrum länger als das erste Wellenlängenspektrum ist,
der erste Polarisator (140a) einen dritten Polarisator (140a-1), der mit der ersten Lichtquelle überlappt ist, und einen vierten Polarisator (140a-2), der mit der zweiten Lichtquelle überlappt ist, enthält, und
eine Polarisationsrichtung des dritten Polarisators und eine Polarisationsrichtung des vierten Polarisators parallel sind, und wobei optional die Polarisationsrichtung des dritten Polarisators und die Polarisationsrichtung des vierten Polarisators senkrecht zu der Polarisationsrichtung des zweiten Polarisators (140b) sind.

## Revendications

1. Capteur de biosignaux (100), comprenant :
une couche transmettant la lumière extensible (110),
une source de lumière (120) sur la couche de transmission de lumière extensible,
un capteur de lumière (130) sur la couche de transmission de lumière extensible,
un premier polariseur (140a) intégré dans la couche de transmission de lumière extensible, le premier polariseur étant configuré pour polariser linéairement la lumière émise par la source de lumière, de sorte que le capteur de biosignaux soit configuré pour amener au moins une partie de la lumière émise par la source de lumière à être polarisée linéairement par le premier polariseur après avoir été émise par la source de lumière, et
un deuxième polariseur (140b) intégré dans la couche de transmission de lumière extensible, le deuxième polariseur étant configuré pour polariser linéairement la lumière reçue au niveau du capteur de lumière, de sorte que le capteur de biosignaux soit configuré pour amener au moins une partie de la lumière reçue au niveau du capteur de lumière à être polarisée linéairement par le deuxième polariseur avant d'être reçue au niveau du capteur de lumière,
ladite couche de transmission de lumière extensible comprenant une pluralité de premières zones (110a) et une seconde zone (110b) entre des premières zones adjacentes de la pluralité de premières zones, ladite pluralité de premières zones comportant chacune un premier module d'élasticité, ladite seconde zone comportant un second module d'élasticité qui est inférieur au premier module d'élasticité,
ladite source de lumière et ledit capteur de lumière se trouvant chacun sur une première zone distincte de la pluralité de premières zones, et
ladite seconde zone étant entièrement raccordée en continu.

2. Capteur de biosignaux (100) de la revendication 1,
ledit premier polariseur (140a) chevauchant au moins partiellement la source de lumière dans une première direction le long de laquelle la source de lumière est configurée pour émettre de la lumière, et
le deuxième polariseur (140b) chevauchant au moins partiellement le capteur de lumière dans la première direction.

3. Capteur de biosignaux (100) d'une quelconque revendication précédente, une direction de polarisation du deuxième polariseur (140b) étant différente d'une direction de polarisation du premier polariseur (140a), et éventuellement un angle entre la direction de polarisation du deuxième polariseur et la direction de polarisation du premier polariseur étant de 30 degrés à 90 degrés.

4. Capteur de biosignaux (100) de la revendication 3, ladite direction de polarisation du deuxième polariseur (140b) étant perpendiculaire à la direction de polarisation du premier polariseur (140a).

5. Capteur de biosignaux (100) d'une quelconque revendication précédente, ledit deuxième polariseur (140b) étant configuré pour bloquer sélectivement la lumière dans une direction de polarisation parallèle à une direction de polarisation de la lumière polarisée linéairement passant à travers le premier polariseur (140a), et transmettre sélectivement la lumière dans une direction de polarisation distincte différente de la direction de polarisation de la lumière polarisée linéairement passant à travers le premier polariseur.

6. Capteur de biosignaux (100) d'une quelconque revendication précédente,
ledit premier polariseur (140a) comprenant une pluralité de premiers motifs s'étendant dans une première direction, et
ledit deuxième polariseur (140b) comprenant une pluralité de seconds motifs s'étendant dans une seconde direction, la seconde direction étant différente de la première direction,
et éventuellement ladite première direction et ladite seconde direction étant perpendiculaires l'une à l'autre.

7. Capteur de biosignaux (100) de la revendication 6,
ladite pluralité de premiers motifs s'étendant dans la première direction et ladite pluralité de seconds motifs s'étendant dans la seconde direction étant intégrés dans la couche transmettant la lumière.

8. Capteur de biosignaux d'une quelconque revendication précédente, comprenant en outre : un retardateur de phase entre la source de lumière et le premier polariseur.

9. Capteur de biosignaux (100) d'une quelconque revendication précédente, ledit capteur de biosignaux étant un capteur de photopléthysmographie, PPG, pouvant être fixé sur la peau.

10. Système de capteur comprenant le capteur de biosignaux (100) d'une quelconque revendication précédente.

11. Réseau de capteurs (500) comprenant le capteur de biosignaux (100) de l'une quelconque des revendications 1 à 9.

12. Réseau de capteurs (500) de la revendication 11,
ledit réseau de capteurs comprenant une pluralité de groupes d'éléments unitaires (510), et chaque groupe d'éléments unitaires de la pluralité de groupes d'éléments unitaires comprenant un biocapteur de l'une quelconque des revendications 1-9,
et éventuellement chaque groupe d'éléments unitaires comprenant en outre un capteur de pression (300).

13. Réseau de capteurs (500) de la revendication 12,
ladite source de lumière (120) comprenant une première source de lumière (120-1) configurée pour émettre de la lumière dans un premier spectre de longueurs d'onde et une seconde source de lumière (120-2) configurée pour émettre
une lumière dans un second spectre de longueurs d'onde, le second spectre de longueurs d'onde étant plus long que le premier spectre de longueurs d'onde,
ledit premier polariseur (140a) comprenant un troisième polariseur (140a-1) chevauchant la première source de lumière, et
un quatrième polariseur (140a-2) chevauchant la seconde source de lumière, et
une direction de polarisation du troisième polariseur et une direction de polarisation du quatrième polariseur étant parallèles, et éventuellement ladite direction de polarisation du troisième polariseur et ladite direction de polarisation du quatrième polariseur étant perpendiculaires à la direction de polarisation du deuxième polariseur (140b).
